# EUROPEAN PATENT APPLICATION

(11) **EP 1 010 426 A1**
(43) Date of publication of application: **21.06.2000**
(21) Application number: 98936672.9
(22) Date of filing: 05.08.1998
(51) Int. Cl.: A61K 31/40, A61N 5/06

(54) **IMMUNOSUPPRESSION BY PHOTOCHEMOTHERAPY**

(30) Priority: 05.08.1997 JP 21039597
(71) Applicant: Meiji Seika Kaisha, Ltd., Chuo-ku, Tokyo 104-0031 (JP)
(72) Inventor: KUROIWA, Yukari Pharmaceutical Research Center, Kouhoku-ku, Kanagawa 222-0002 (JP); ARAAKE, Minako Pharmaceutical Research Center, Kouhoku-ku, Kanagawa 222-0002 (JP); AIZAWA, Katsuo Tokyo Medical College, Tokyo 160-0022 (JP)
(74) Representative: Maureau, Philippe
(86) International application number: JP9803492
(87) International publication number: WO9907364

(57) **Abstract**

Immunosuppressive compositions for photochemotherapy which contain as the active ingredient tetrapyrrole derivatives represented by general formula (I) or pharmacologically acceptable salts thereof wherein n is 1 or 2. Administration of the derivatives of general formula (I) or salts thereof as immunosuppressive agents in photochemotherapy makes it possible to prevent or treat abnormally supernumerary immune responses, for example, contact hypersensitivity or implant rejection reactions.

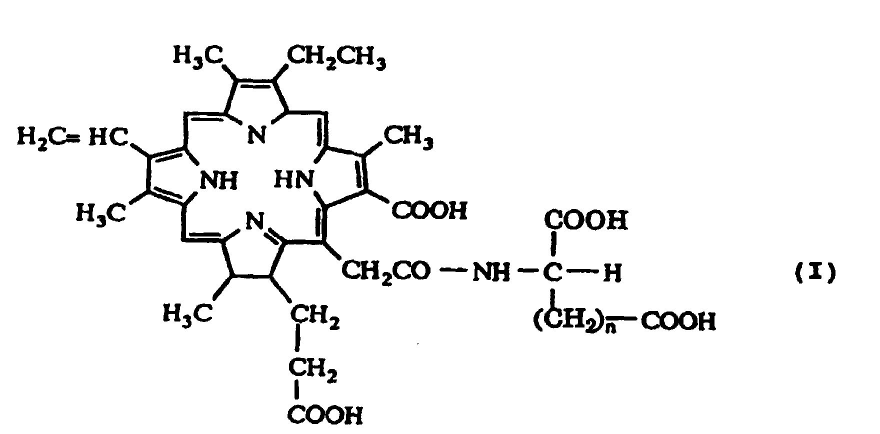

## Description

### Technical Field

This invention relates to a novel immunosuppressant composition to be used in photochemotherapy. The immunosuppressant composition according to this invention contains a fluorescent tetrapyrrole derivative or a salt thereof as an active ingredient. This invention further relates to a novel photochemotherapeutical method for suppressing an hyperimmune response, which comprise administering a fluorescent tetrapyrrole derivative or a salt thereof as an immunosuppressant, followed by irradiating the administered derivative with a laser light.

### Background Art

A photochemotherapeutical method is a method of therapeutically treating a disease by the administration of a photosensitive substance capable of being excited by an ultraviolet ray or a laser light. In this method, the therapeutic effect is obtained by applying an irradiation of a light either to a part or parts of such tissue of a living body of the patient where said photosensitive substance as pre-administered is present and accumulated, or to a flow of intracorporeal blood containing said photosensitive substance, or to a flow of blood as formed by extracorporeal circulation of the blood containing said photosensitive substance, or otherwise, so that said photosensitive substance is photochemically excited to display the therapeutic effect.

Japanese Patent Publications Kokoku Hei-6-88902 and Hei-6-89000 and their corresponding United States Patent specifications No. 4,675,338 and No.4,693,885 disclose fluorescent tetrapyrrole derivatives in which at least one carboxyl group of a certain tetrapyrrole compound having a plural number of side chains in the form of the carboxyl group or carboxylic acid residue has been condensed via one or more amido-linkages with the amino group of an amino-dicarboxylic acid of 4 to 10 carbon atoms, for example, aspartic acid or glutamic acid, and which derivatives may be exemplified as mono-L-aspartyl chlorin e6 and mono-L-glutamyl chlorin e6, or salts thereof. There is disclosed the uses of these tetrapyrrole derivatives as a photochemotherapeutic agent for the purposes of diagnosis and therapy of tumors. The above Japanese patent publications and U. S. patent specifications further disclose that the said fluorescent tetrapyrrole derivative accumulated within the tumor tissues after its administration can be excited photochemically by being irradiated with a strong light, for example, a laser beam and can thereby display the effect to kill the tumor cells.

With respect to the photochemotherapy, the prior art further reports, in addition to the above-mentioned therapy for cancers, that the photochemotherapy is also applicable to the method of closing neovascular vessels in choroideae of eyes (refer to Japanese Patent First Publication KOKAI No.Hei-9-71531 and U.S. Patent specification No.5,633,275).

Further, U. S. Patent No.5,028,594 discloses the application of photochemotherapy to a method for the selective removal of hemopoietic cells participating in rheumatoid arthritis. On the other hand, Kenneath et. al. propose a method for a therapeutic treatment of proliferative arthropathia by photochemotherapy, which comprises involving destruction of synovial cells (see PCT Application International Published specification WO94/17797).

In vivo, in general, an immune response can occur against an invasion of a non-self foreign substance into the living body, whereby the homeostatis of organisms is maintained. In some instances, however, the immune response can take place very much excessive, that is, there may occur hyperimmune responses which result in some injury in the living organisms. Such injuriously occuring hyperimmune responses may, for example, be the autoimmune diseases or the rejections reactions occurring in the organ transplantation. In such cases, there may be required a therapeutic method of regulating such hyperimmune responses abnormally occurring in the organisms. Drugs developed for such purposes is an immunomodulator in a broad sense. The immunomodulator in the broad sense is classified generally into three classes.

That is to say, the immunomodulators include an immunopotentiator, an immunomodulator of a narrow sense, and also an immunosuppressant. The immunopotentiator is an agent capable of potentiating the immune response and is used for treating malignant tumors, infectious diseases, and so on. The immunomodulator of the narrow sense is the drug for correcting such abnormality of the immune response as involved in the organisms, and it is used for curing the autoimmune diseases and various allergies. The immunosuppressant is the drug for suppressing an immune over-response or hyperimmune response and is used for treating such diseases as autoimmune diseases, malignant tomors, and a rejection reaction to a graft as transplanted in the organ transplantation (see "Therapeutics" written by Nobuyuki Miyasaka, Vol.26, No.2, pp.15-18).

Further, an allergy may be classified into ones of I type to IV type, according to the classification of Coombs and Gell. Allergy of the IV type includes a contact hypersensitivity, Tuberculin reaction, a granulomatous hypersensitivity and others.

Hypersensitivity which is belonging to the IV type allergy can take place not by a humoral immunity, but by a cell-mediated immunity ( "Immunology Illustrated" , written by Ivan Roitt Jonathan Brostoff David Male, The original Third edition, translated in Japanese under supervision of Tomio Tada, published by Nanko-do, pp.325-336). Contact hypersensitivity includes an eczematoid reaction with itching, which appears on the skin where an allergen has been adhered to the skin. In such eczematoid reaction, a chemical substance or substances having a molecular weight of 1000 or lower can act as the allergen which forms a combined material with a cell membrane protein of the epidermic cell, so that the information of the allergen may be transmitted to T-cells through Langerhans' cells and thus there occurs the contact hypersensitivity via the T-cells activation. For treating the allergic contact dermatitis, which is a typical contact hypersensitivity, there have been used a glucocorticoid, adrenocorticotropic hormone (ACTH) and the like, as a non-specific immunosuppressant.

Recently, on the other hand, there have extensively been carried out surgical, organ transplantations with various internal organs, including kidney. In such organ transplantation, the rejection reaction occurs. In order to suppress such rejection reaction, there has been made irradiation of radio-active radiations or administration of an immunosuppressant such as cyclosporin, FK506, and the like. However, it has been known that such known methods available for the immunity suppression can occasionally lead to heavy opportunistic infectious diseases by infectibility and can lead to side reactions such as myelonic suppression, and so on (see "Therapeutics" written by Mitsuo Homma, Vol.26, No.2 pp.10-13).

As described above, there have been reported various applications of the photochemotherapy to destruction of tumor cells, removal of hemopoietic cells, destruction of proliferative synovial cells causative for articular rheumatism and occulusion of neovascular vessels. Until now, however, there is no announcement as to that the photochemotherapy would be applicable for the purpose of achieving the immunity suppression.

An object of this invention is to develope a photochemotherapeutical method which is suited for the prevention or therapeutic treatment of a contact hypersensitivity and also suited for the prevention or therapeutic treatment of rejection reactions occurring in the organ transplantation. A further object of this invention is to provide a novel immunosuppressant which is suitable to be used in the applications of the photochemotherapeutical method as a means for the suppression of immunity.

Another object of this invention will become clear in the descriptions given hereinafter.

### Disclosure of Invention

We, the inventors of this invention, have proceeded our investigations in order to develope a photochemotherapeutical method which is applicable to the prevention or treatment of a contact hypersensitivity and to the prevention or treatment of rejection reactions occurring in the organ transplantation, and also in order to search for a medicine capable of being excited with a light and thus capable of exhibiting an effect of suppressing the immunity. As a result, when a photosensitive substance, mono-L-aspartyl chlorin e6 tetra-sodium salt, which is described in the Japanese Patent Publications KOKOKU Hei-6-88902 and Hei-6-89000 and U. S. patent specifications No.4,675,338 and No.4,693,885 referred to above, and which has been examined in clinical testing for a photochemotherapy of malignant tumors, is employed in a photochemotherapeutical method in such a way that the compound to be tested is intravenously administered to such mice inherently capable of causing a contact hypersensitivity, and then the circulating blood stream in the blood vessel under the skin is irradiated with a laser light at 664 nm, we have now found that the compound under test exhibits an immunosuppressive effect in that the swelling of an ear of the mice normally observable in the testings of a contact hypersensitivity can remarkably be suppressed; and also we have now found that the mice having received such same photochemotherapeutical method as above can exhibit the immunosuppressive effect that the rejection reactions possibly raised in the transplantation of skin between mice of different inheritance characters can be preventively suppressed. Further, mono-L-glutamyl chlorin e6 tetra-sodium salt can also be expected to exhibit the immuno-suppressive effect similar to the above.

Mono-L-aspartyl chlorin e6 and mono-L-glutamyl chlorin e6 are the compounds which can be prepared by the process for the preparation of them as described in Example 19 and Example 21, respectively, of U. S. Patent specification No.4,675,338. According to the recent investigation, mono-L-aspartyl chlorin e6 has been recognized to be a substance having a chemical structure represented by the following formula (A)

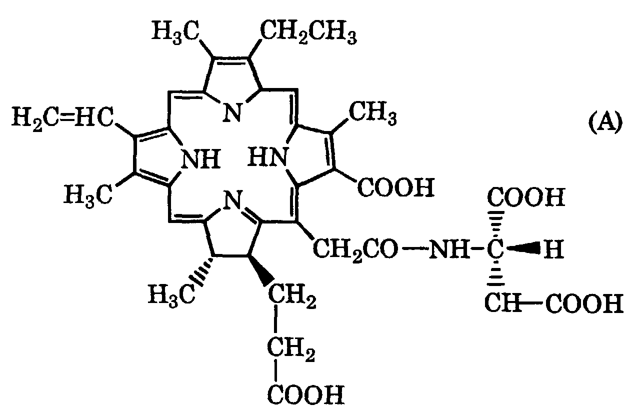

It has also been found that mono-L-glutamyl chlorin e6 is a substance having a chemical structure represented by the following formula (B)

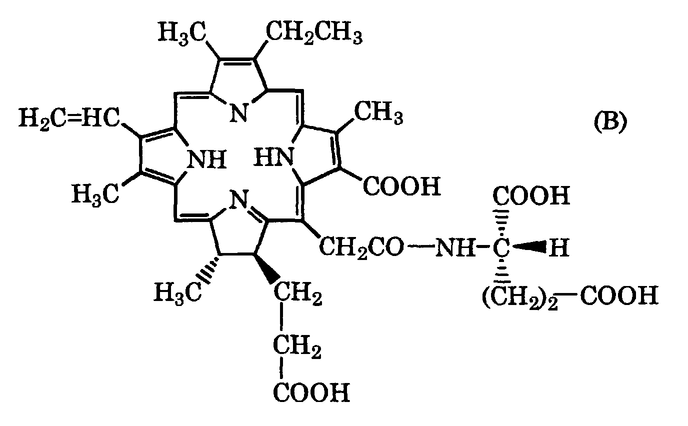

Further, it has been found that in general, a fluorescent tetrapyrrole derivative represented by the undermentioned general formula (I) or a pharmacologically acceptable salt thereof, when used in the photochemotherapeutical method, can exhibit an immunosuppressive effect. This invention has been completed on the basis of these findings.

According to a first aspect of this invention, therefore, there is provided an immunosuppressant composition for use in a photochemotherapeutical method, characterized in that the composition contains as an active ingredient a fluorescent tetrapyrrole derivative represented by the following general formula (I) 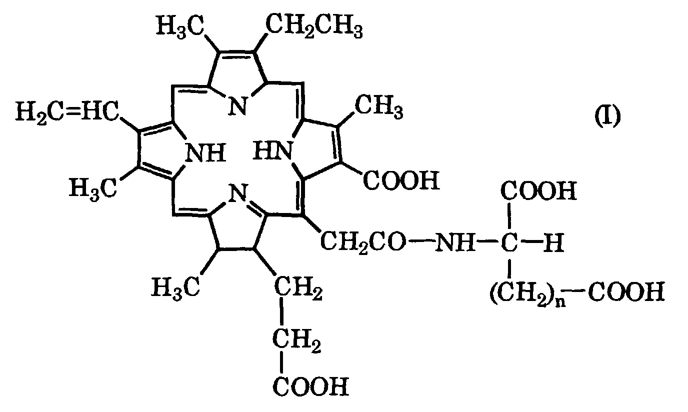 wherein n stands for an integer of 1 or 2, or a pharmacologically acceptable salt thereof, and that the composition further contains a pharmacologically acceptable carrier or carriers, in admixture with the active ingredient.

The tetrapyrrole derivatives of general formula (I) used in the immunosuppressant composition according to this invention are preferred to be those having the configuration represented by the following general formula (II) 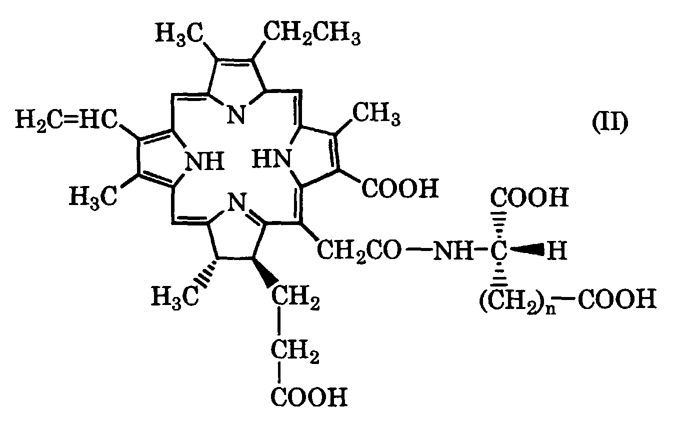 wherein n stands for an integer of 1 or 2.

Of the compounds having the general formula (II) above, the compound where n = 1 is such one having the structure of the formula (II) above wherein the amino group of the L-aspartic acid is combined in the form of an amido-linkage with the acetyl group -CH₂COOH as one side-chain of the tetrapyrrole ring shown in the formula above. This compound is mono-L-aspartyl chlorin e6. It is preferable that this compound is used in the form of the tetra-sodium salt at the four carboxyl groups thereof.

Of the compounds having the general formula (II), the compound where n = 2 is such one having the structure that in place of the L-aspartic acid , L-glutamic acid is combined through the amido-linkage. This compound is mono-L-glutamyl chlorin e6.

The compounds of general formula (I) or general formula (II) usable in this invention have the four carboxyl groups in their molecule and can generally form a pharmacologically acceptable salt in combination with a base. As their salts formed by reacting with a base, there may be exemplified those with sodium, potassium, calcium, magnesium and ammonium, as well as addition salts with triethylamine, trimethylamine, morpholine and piperidine.

The immunosuppressant composition according to the first aspect of this invention is effective for the prevention or therapeutic treatment of diseases which are relating to the hyperimmune responses, for example, a contact hypersensitivity, a tuberculin reaction, and a granulomatous hypersensitivity. This composition of this invention is also effective for the prevention or lowering of the rejection reactions possibly raised after the organ transplantation of various organs or transplantation of skin.

The compounds of general formula (I) or general formula (II) or their salts contained as the active ingredient in the composition according to the first aspect of this invention may be administered orally or may be administered parenterally by intravenous injection or intramuscular injection or by intrarectal administration. The compounds above-mentioned may also be administered percutaneously. For instance, the composition according to this invention may be formulated in the form of a preparation comprising the compound of general formula (I), preferably the compound of general formula (II), in the form of tetra-sodium salt thereof. The composition may be formulated into a lyophilized and sterilized preparation containing no pyrogenic substance.

In the composition according to this invention which is suited for oral administration, the compound of general formula (I) or a salt thereof as the active ingredient may be present in admixture with a solid or liquid carrier or carriers and may be formulated in the form of tablets, buccal tablets, troches, capsules, suspension, syrup, and the like.

The content of the compound of general formula (I) or general formula (II) as the active ingredient in the composition according to the first aspect of this invention may vary depending upon the form of the preparation intended. A convenient content of the compound above-mentioned may be in the range of about 2-60 % based on the weight of a dosage unit of the administrable preparation of the composition.

A preferred form of the injectionable preparations of the composition according to the first aspect of this invention is a sterile aqueous solution or dispersion or a sterile lyophilized preparation containing the compound of general formula (I) as the active ingredient. As a preferred liquid carrier to be used here, there may be exemplified water, ethanol, glycerol, propylene glycol, vegetable oils and the like. In most cases, the injectionable preparations may preferably contain further an isotonic agent, for example, sugar or sodium chloride.

Where the composition according to the first aspect of this invention is to be formulated in the form of an injection preparation, it is possible to incorporate additionally therein an agent for delaying the absorption of the compound of general formula (I) as the active ingredient, for example, aluminium monostearate or gelatin.

The dosage of the compound of general formula (I) or a salt thereof, which is contained in the immunosuppressant composition according to the first aspect of this invention, may vary depending upon the purpose of the therapeutical treatment and the degree of symptom of the disease, and it may generally be 0.2-10 mg of the said compound per day once for adult patients. The optimum dosage may be determined by a suitable preliminary testing which is made by those skilled in the art.

After the administration of the pharmaceutical composition according to the first aspect of this invention, the compound of formula (I) or a salt thereof presented in the blood stream of the patient is excited photochemically by irradiation with a laser light of a wave length in the range of 620-760 nm. As the irradiation sources for the laser light used, there may be utilized a powerful continuous laser beam source equipped with optical filters, or an excited pigment or other laser beam-feeding system. Among the available irradiation sources of laser light as above-mentioned, it is desirable to use such a laser source which can generate a laser beam at a full output power of at least 500 mW, at a radiation intensity of 10-100 mW/cm² and at a wave length of 620-760 nm. At present, some of commercially available laser generators can satisfy the requisites for the laser-generation as given above.

The acute toxicity of mono-L-aspartyl chlorin e6 tetra-sodium salt which is one example of the compounds having the above general formula (I) was 164 mg/kg for its LD₅₀ value when tested in CD-1 mice (male). Further, in a photo-toxicity test of mono-L-aspartyl chlorin e6 tetra-sodium salt, it has been found that this particular compound shows no reactions such as erythema, edema, etc. and therefore that this compound is a highly safe compound.

According to a second aspect of this invention, there is provided a photochemotherapeutical method for the prevention or therapeutic treatment of a hyperimmune response, which comprises administering orally or parenterally a therapeutically effective amount of a fluorescent tetrapyrrole derivative of general formula (I) shown above or a pharmacologically acceptable salt thereof, preferably mono-L-aspartyl chlorin e6 or mono-L-glutamyl chlorin e6 or a pharmacologically acceptable salt thereof, to a human or mammal susceptible to a hyperimmune response or an immune over-response, irradiating the blood stream flowing in the blood vessel and having contained the so administered compound with a laser light, thereby to expose said tetrapyrrole derivative of general formula (I) or a salt thereof contained in the flowing blood stream to the irradiating laser light and thus to excite photochemically said compound present in the blood, with bringing about the generation of active oxygen, and thereby decreasing or suppressing development of the immunity by means of the active oxygen.

In the therapeutical method according to the second aspect of this invention, the tetrapyrrole derivative of general formula (I), preferably mono-L-aspartyl chlorin e6 or mono-L-glutamyl chlorin e6 or a salt thereof, when administered parenterally, may be administered by intravenous or intramuscular injection or per rectum. It may also be administered orally. The dose for the administration of the compound is to be an effective amount thereof which is sufficient to excite photochemically the administetered tetrapyrrole derivative, particularly mono-L-aspartyl chlorin e6 or mono-L-glutamyl chlorin e6 or a salt thereof present in the blood stream and thereby to cause the compound to generate the active oxygen.

The laser light to be irradiated may be irradiated as its beam onto the whole body or various parts of the patient, for example, the abdominal region, the leg region or the hand region over the skin thereof. Thus, the irradiation of the laser light may be effected in a manner that the laser light can transmit through the skin layer and through the under-lying blood vessel wall layer, so that the laser light can reach the blood stream circulating in the blood vessels under the skin. The laser light irradiation may also be effected to such blood stream which are being circulated extracorporeally.

The number of times of the irradiation of laser light may be one or more. The combination of the dosage administered of the compound with the number of times of the irradiation of the laser light may be such one that said combination is to be sufficient to control the immunity, and thus to give the therapeutical effect intended. The dosage of the compound of general formula (I) as given and the number of times of the irradiation of the laser light required may easily be decided by preliminary tests by those skilled in the art, depending on the therapeutical purposes. The laser light irradiated may preferably be such one having wave lengths of 620-760 nm and may be irradiated at a radiation intensity in the range of 10-100 mW/cm².

The photochemotherapeutical method according to the second aspect of this invention is effective for the prevention or therapeutic treatment of a contact hypersensitivity. The method according to the second aspect of this invention may also be effective for the prevention or treatment of the rejection reactions to a graft employed in the transplantation thereof.

According to a further aspect of this invention, there is provided a use of the tetrapyrrole derivative of general formula (I) above-mentioned or a pharmacologically acceptable salt thereof in the preparation of such an immunosuppressant composition which is to be used for preventing or treating photochemotherapeutically a hyperimmune response.

### Best Mode for Carrying Out the Invention

This invention is now illustrated in detail with reference to the following Test Examples and Examples, to which this invention is not limited.

### Test Example 1

(i) Mono-L-aspartyl chlorin e6 tetra-sodium salt was dissolved in a physiological saline solution at a concentration of 20 mg/ml, and the resultant solution was sterilized to prepare an injectionable solution. The solution was intravenously injected to each group of BALB/c mice of 10 weeks-old (5 mice per group) in such a manner that different volumes of the injectable solution as prepared were injected so as to adjust the dose of the compound under test, namaly, mono-L-aspartyl chlorin e6 tetra-sodium salt, to 0.001 mg/kg, 0.05 mg/kg, 0.01 mg/kg or 0.1 mg/kg for the respective mice group. After the intravenous injection, hair of the abdominal side of the mice was shaved off. Thirty minutes after the administration of the test compound, a laser light at a wave length of 664 nm was irradiated onto the exposed skin of the abdominal side of the mice to give a total fluence of 10 J/cm² under the conditions of the laser output of 20 mw/cm² and the radiated spots of a diameter of 18 mm. Thus, the irradiating laser light was received by mono-L-aspartyl chlorin e6 tetra-sodium salt which had been presented in the blood vessels lying under the skin of the abdominal side of the mice, so that the compound was photochemically excited.
(ii) Twenty-four hours after the administration of mono-L-aspartyl chlorin e6 tetra-sodium salt, the cervical part, both the armpits and both the groins of each mouse having been irradiated with the laser light (5 mice per group) were coated thereon with an aqueous solution containing 5 % picryl chloride acting as an immune sensitizer over the skin to effect the priming (the primary sensitization).
   Five days after the primary sensitization, an aqueous solution containing 1 % picryl chloride was applied to one ear of each mouse under test, to effect the secondary sensitization. Thus, there were prepared the treated groups of the test mice having a contact hypersensitibity.
(iii) Twenty-four hours after the secondary sensitization, namely six days after the primary sensitization, measurements were made of the thickness of both the ears of each mouse under test by a thickness gauge, and the increases in the thickness of ears were calculated from the difference which was found between the thickness of the one ear having not received the application of picryl chloride and the thickness of the another ear having received the application of picryl chloride for the secondary sensitization. Then, the rates(%) of ear swelling of each mouse of the treated group of mice were evaluated, on such assumption that the average value of the increase in the thickness of the secondarily sensitized ears of the mice of the untreated group was regarded as amounting to 100 %, in respect of the mice of the untreated group of mice where the mice were neither administered with the test compound, nor were irradiated with the laser light, but where the mice received only the primary and secondary sensitizations with picryl chloride.

The evaluated rates (%) of the ear swelling for each group of the mice, either untreated or treated, are shown in the following Table 1.

**Table 1**

| Dosage(mg/kg)of the compound tested | | Rate of Ear swelling, (%) (Average value ± Standard deviation) |
|---|---|---|
| 0 | (Untreated group) | 100 |
| 0.001 | (Treated group) | 54.4 ± 7.2 |
| 0.01 | (Treated group) | 53.2 ± 20.6 |
| 0.05 | (Treated group) | 33.3 ± 4.4 |
| 0.1 | (Treated group) | 27.8 ± 8.5 |

As is clear from the results of test given in Table 1, it is observed that the photochemotherapeutical method comprising administering mono-L-aspartyl chlorin e6 tetra-sodium salt by the intravenous injection of it in dosages of 0.001 mg/kg or more and then irradiating the administered test compound with a laser light is able to suppress by 45% or more the ear swelling of the mice of the treated group which had received the primary and secondary sensitizations with picryl chloride. The above-mentioned tests for measuring the rates of ear swelling, which comprise subjecting the mice to the primary and secondary sensitizations with picryl chloride as an allergen, are a typical experiment for simulating to the contact hypersensitivity. Thus, the fact that the above-mentioned tests for the photochemotherapeutical method comprising administering mono-L-aspartyl chlorin e6 tetra-sodium salt by intravenous injection and then irradiating the dosed compound with a laser light was able to suppress the rate of ear swelling to a significant extent, is to reveal that the hyperimmune response could be suppressed significantly by said method.

### Test Example 2

The following experiment was conducted in order to evaluate the therapeutic effect of the photochemotherapeutical method of this invention for treating the rejection reactions raised upon the transplantation of skin in mice.

Thus, as the mice (the recipient) which were to recieve a transplantation of skin, were used mice of BALB/c (H-2K^{d}) strain (ten mice per group). One day before the skin transplantation, mono-L-aspartyl chlorin e6 tetra-sodium salt as the test compound was administered to the recipient mice intravenously at a dose of 1 mg/kg and then the hair of the abdominal side of each mouse of the recipient was shaved off. Thirty minutes after the intravenous injection of the test compound, a laser light at the wave length of 664nm was irradiated onto the abdominal side of each recipient mouse at the laser output of 20 mW/cm² , at the irradiated spots of a diameter of 18 mm and at a total laser fluence of 10 J/cm².

On the other hand, mice of CH/HeN(H-2K^{b}) strain were used as the mice (the donor) which were to afford the graft of skin for the transplantation to the recipient. The graft of skin to be used for the skin transplantation was one piece of the tail skin of the donor mice which was cut into a piece of about 5mm x 5 mm. This graft of skin was transplanted on the back side of the recipient mouse in such a manner that hair of the back side of the mouse was firstly shaved off, then one region of the skin of the hair-shaved back side of mouse was stripped off and the skin graft was transplanted onto the skin-stripped region of the back side of the recipient mouse. This skin transplantation was performed twenty-four hours after the irradiation of laser light on the recipient mouse.

Then, observation was made as to whether the skin graft so transplanted would fall off or not fall from the recipient mouse within the period of from the time of the skin transplantation to the 15 the days after the intravenous injection of the test compound.

The number of the recipient mice which normally have the transplanted and surviving skin graft remaining on the back side of mouse was counted at each of the given days of observation. The ratio of the number of the graft-remaining mouse at the each day to the number of the mouse at 0 day was expressed as percentage (%), with that the number of the recipient mice at the 0 day being assumed as 100.

Mice of the untreated group were those mice which received the skin transplantation only but neither received the administration of the test compound nor received the irradiation of laser light.

The results of the tests as obtained are given in the following Table 2.

**Table 2**

| Elapse Day (Day) | Percentage (%) of number of the mice with the surviving and remaining skin graft transplanted | |
|---|---|---|
| | Control group (Untreated group) | Treated group (having received administration of test compound) |
| 0th (The day of skin transplantation) | 100 | 100 |
| 6th | 50 | 100 |
| 7th | 50 | 80 |
| 10th | 0 | 80 |
| 11th | 0 | 80 |
| 15th | 0 | 0 |

The falling off of the skin graft as transplanted is owing to an excessive occurrence of the immune rejection reactions in the recipient mice. As is clear from the test results given in Table 2, the recipient mice (of the treated group), which have received the above-mentioned photochemotherapeutical treatment comprising the administration of mono-L-aspartyl chlorin e6 tetra-sodium salt and the irradiation of laser light, show that only 20 % of the total mice tested could involve the falling off of the skin graft within the period of 6 th to 11 th days. In contrast, the mice of the control group (the untreated group) show that all the untreated mice tested could involve the falling off of the skin graft once transplanted. It is therefore clear that the photochemotherapeutical method effected in this Test Example can significantly suppress the rejection reactions which would raise in the transplantation of the graft of skin.

The following Examples illustrate some exemplary formulations of the immunosuppressant composition according to this invention.

### Example 1

The following ingredients were admixed with each other in the following proportions (by weight) to prepare a base powder.

| | |
|---|---|
| Sucrose | 80.3 g |
| Tapioca starch | 13.2 g |
| Magnesium stearate | 4.4 g |

The base powder so prepared was mixed with an appropriate amount of mono-L-aspartyl chlorin e6 tetra-sodium salt, and the resulting mixture was pressed into tablets in a conventional manner to give tablets each containing 100 mg of mono-L-aspartyl chlorin e6 tetra-sodium salt as the active ingredient.

### Example 2

Mono-L-aspartyl chlorin e6 tetra-sodium salt (200 mg) was dissolved in a physiological saline solution to give a final concentration of 20 mg/ml of the tetra-sodium salt. The resulting solution was subjected to a sterilizing treatment to prepare an injectable solution. This injectable solution is suitable for intravenous and intramuscular administrations.

### Industrial Applicability

As explained above, this invention relates to a photochemo- therapeutical method comprising administering to human or mammals susceptible to a hyperimmune response a tetrapyrrole derivative of the general formula (I) above, preferably mono- L-aspartyl or mono-L-glutamyl chlorin e6 or a salt thereof, and irradiating the blood stream having contained the dosed compound with a laser light. The method of this invention is effective to prevent or treat photochemotherapeuticaly various immune diseases which can be caused due to the hyperimmune response, and it is of a high safety.

## Claims

1. An immunosuppressant composition for use in a photochemotherapeutical method, characterized in that the composition contains as an active ingredient a fluorescent tetrapyrrole derivative represented by the following general formula (I) 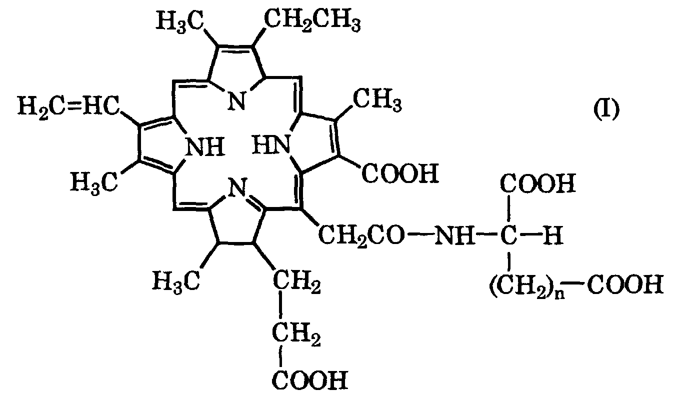 wherein n stands for an integer of 1 or 2, or a pharmacologically acceptable salt thereof, in admixture with a pharmacologically acceptable carrier or carriers.

2. A composition according to Claim 1, wherein the tetrapyrrole derivative of general formula (I) is a stereo-isomer represented by general formula (II) 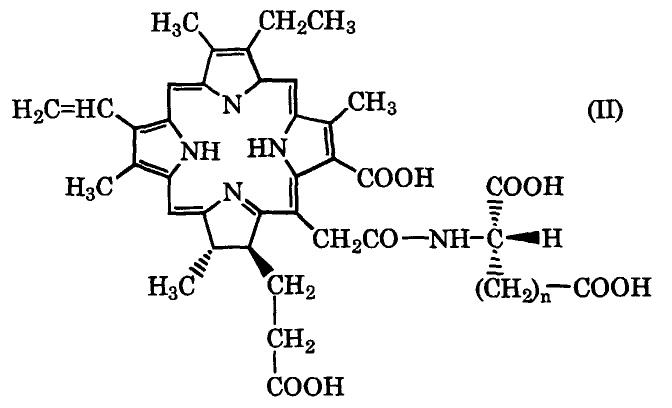 wherein n stands for an integer of 1 or 2.

3. A composition according to Claim 1 or 2, wherein the compound of general formula (I) or general formula (II) or a salt thereof is mono-L-aspartyl chlorin e6 or tetra-sodium salt thereof.

4. A composition according to Claim 1, wherein the immune suppression is applied to the prevention or therapeutic treatment of a contact hypersensitivity.

5. A composition according to Claim 1, wherein the immune suppression is applied to the prevention or therapeutic treatment of a rejection response to a graft.

6. A photochemotherapeutical method for the prevention or therapeutic treatment of a hyperimmune response, which comprises administering orally or parenterally a therapeutically effective amount of a fluorescent tetrapyrrole derivative of general formula (I) shown in Claim 1 or a pharmacologically acceptable salt thereof, preferably mono- L-aspartyl chlorin e6 or mono-L-glutamyl chlorin e6 or a pharmacologically acceptable salt thereof, to a human or mammal susceptible to a hyperimmune response, irradiating the blood stream flowing in the blood vessel and having contained the so administered compound with a laser light, thereby to expose said tetrapyrrole derivative of general formula (I) or a salt thereof contained in the flowing blood stream to the irradiating laser light and thus to excite photochemically said compound present in the blood, with bringing about the generation of active oxygen, and thereby decreasing or suppressing development of the immunity by means of the active oxygen.

7. A method according to Claim 6, wherein the tetrapyrrole derivative administered is mono-L-aspartyl chlorin e6 or tetra-sodium salt thereof.

8. A method according to Claim 6, wherein the tetrapyrrole derivative administered is mono-L-glutamyl chlorin e6 or tetra-sodium salt thereof.

9. A method according to Claim 6, wherein the laser light irradiated is a laser light having a wave length in the range of 620-760 nm.

10. A method according to Claim 6, wherein the immune response to be prevented or treated is a contact hypersensitivity.

11. A method according to Claim 6, wherein the immune response to be prevented or treated is a rejection reaction to a transplanted graft.

12. Use of a tetrapyrrole derivative of general formula (I) shown in Claim 1 or a pharmacologically acceptable salt thereof, in the preparation of such an immunosuppressant composition which is to be used for photochemotherapeutically preventing or treating a hyperimmune response.
